## Europäisches Patentamt
## European Patent Office
## Office européen des brevets

(19)

(11) **EP 0 354 316 B1**

(12) # EUROPÄISCHE PATENTSCHRIFT

(45) Veröffentlichungstag und Bekanntmachung des Hinweises auf die Patenterteilung:
**01.05.1996 Patentblatt 1996/18**

(51) Int Cl.[6]: **C10G 25/05**, C07C 7/13

(21) Anmeldenummer: **89110336.8**

(22) Anmeldetag: **08.06.1989**

(54) **Verfahren zur Feinentschwefelung von Kohlenwasserstoffen**

Process for the deep desulphurisation of hydrocarbons

Procédé de désulfuration poussée d'hydrocarbures

(84) Benannte Vertragsstaaten:
**AT BE DE ES FR GB IT NL SE**

(30) Priorität: **23.07.1988 DE 3825169**

(43) Veröffentlichungstag der Anmeldung:
**14.02.1990 Patentblatt 1990/07**

(73) Patentinhaber: **HÜLS AKTIENGESELLSCHAFT**
**D-45764 Marl (DE)**

(72) Erfinder:
- **Wildt, Thomas, Dr.**
  **D-4300 Essen 1 (DE)**
- **Nierlich, Franz, Dr.**
  **D-4370 Marl (DE)**
- **Droste, Wilhelm, Dr.**
  **D-4370 Marl (DE)**
- **Neumeister, Joachim, Dr.**
  **D-4370 Marl (DE)**
- **Scholz, Bernhard, Dr.**
  **D-4370 Marl-Polsum (DE)**

(56) Entgegenhaltungen:
**EP-A- 0 056 197**     **EP-A- 0 064 464**
**GB-A- 1 168 822**     **US-A- 4 188 285**

- **CHEMICAL ABSTRACTS, Band 95, Nr. 7, Oktober 1981, Seite 159, Zusammenfassung Nr. 118166y, Columbus, Ohio, US;**
- **CHEMICAL ABSTRACTS, Band 95, Nr. 24, Dezember 1981, Seite 155, Zusammenfassung Nr. 206517u, Columbus, Ohio, US;**

EP 0 354 316 B1

**Beschreibung**

Die Erfindung betrifft ein Verfahren zur Feinentschwefelung von Kohlenwasserstoffen, die Sulfide, Disulfide, Trisulfide oder Tetrasulfide oder deren Mischungen enthalten.

Schwefelverbindungen sind allgemein bekannt als Katalysatorgifte, besonders für Katalysatoren, die Übergangsmetalle der Gruppe 8 enthalten. Aufgrund ihres oft unangenehmen Geruches sind sie darüber hinaus z. B. in Propan und Butanen unerwünscht, die zunehmend Verwendung im Aerosolbereich finden. Selbst Schwefelgehalte, die weit unter 0,1 ppm liegen, können hier noch als sehr störend empfunden werden.

Insbesondere die Entfernung von Di-, Tri und höheren Oligosulfiden ist deshalb wichtig, da viele petrochemisch gewonnene Kohlenwasserstoffe, bedingt durch ihre Vorreinigung, z. B. nach dem Merox-Verfahren gemäß EP-A 0 235 462 diese Verbindungen enthalten.

Es ist bekannt, Sulfide und Disulfide durch Adsorption zu entfernen. Häufig untersuchte Adsorptionsmittel sind z. B. Aktivkohle, Zeolithe und poröse Silikate.

N. Gryazev et al. beschreiben die Adsorption von Suldifen und Disulfiden aus Lösungen an Zeolithen, Silikagel und Aluminosilikaten (Khim. Seraorg. Soedin., Soderzh. Neft. Nefteprod., 9 (1972), 415 - 420). S. Tanada und K. Boki untersuchten ebenfalls die Entfernung von Schwefelverbindungen an Zeolithen und Silikaten, darüber hinaus aber noch Aktivkohle für die Adsorption von Dimethylsulfid (Tokushima Bunri Daigaku Kenkyu Kiyo, 15 (1976), 33 - 38).

I. P. Muklenov et al. entfernten Dimethylsulfid und Dimethyldisulfid neben weiteren Verbindungen durch Adsorption an Aktivkohle aus Abgasen (Bum. Prom.-st. 7 (1978), 27 - 28).

Gemäß N. A. Sankhin et al., Bum. Prom-st. 11 (1979), 27 - 28, ist es ebenfalls bekannt, Dimethylsulfid und Dimethyldisulfid neben weiteren Schwefelverbindungen, die bei der Herstellung von Sulfaten anfallen, an Aktivkohle zu adsorbieren.

Auch T. Suetaka und M. Munemori beschreiben die Adsorption von Sulfiden, und zwar von Dimethylsulfid und Diethylsulfid, an Aktivkohle (Akushu no Kenkyn 18, 72 (1987), 32 - 34.

Aus Shcherbina et al., Obshch. Prikl. Khim., No. 5 (1972), 113 - 114, ist die Adsorption von Schwefelverbindungen an Natrium-Zeolithen vom X-Typ und aus Mikhal'skaya et al., Khim. Khim. Tekhnol. 9 (1975), 64 - 66, an Natriun-Zeolithen vom Y-Typ bekannt. Mit Hilfe von Natrium-Zeolithen von X-Typ lassen sich Disulfide ebenso wie Mercaptane aus Kohlenwasserstoffen entfernen, während dies bei Sulfiden nur unvollständig geschehen soll.

JP-OS 59/160 584 beschreibt die Adsorption von Disulfiden und Sulfiden neben H$_2$S, COS und Mercaptanen an schwermetallhaltiger Aktivkohle.

Im weiteren ist es aus DD-PS 241 196 und DD-PS 241 197 bekannt, Schwefelwasserstoff und organische Schwefelverbindungen aus Kohlendioxidhaltigen Gasgemischen durch Adsorption an Zink- und Mangan-Zeolithen des X-Typs zu entfernen.

Hierbei betrifft DD-PS 241 196 ein Verfahren zur Verhinderung der Kohlenoxisulfidbildung in Entschwefelungsprozessen und DD-PS 241 197 in sorptiven Trennprozessen.

EP-A-0 056 197 und US-A-4 188 285 beschreiben die Adsorption von organischen Schwefel verbindungen aus Kohlenwasserstoffen durch Adsorption an Zink- und Silber-Zeolithen. EP-A-0 064 464 beschreibt die Entfernung von Schwefelverbindungen durch Adsorption an Kupfer- und Zink-Zeolithen der X- und Y- Typen.

Ziel dieser Verfahren ist es u. a., den Schwefelverlust infolge Kohlenoxisulfid-Bildung für die nachgeschaltete Elementarschwefelgewinnung zu senken.

Beide Verfahren können nur in dem sehr eingeschränkten Temperaturbereich von 10 bis 50° C ausgeübt werden.

Gemäß DD-PS 241 197 ist eine thermische Regenerierung der mit Schwefelverbindungen beladenen Zeolithe möglich.

Alle bisher für die Adsorption von Sulfiden und Disulfiden beschriebenen Verfahren eignen sich nicht für die Feinentschwefelung von Kohlenwasserstoffen.

Der Erfindung liegt daher die Aufgabe zugrunde, ein Verfahren zur Feinentschwefelung von Kohlenwasserstoffen, die Sulfide, Disulfide, Trisulfide, Tetrasulfide und weitere Polysulfide enthalten, zu entwickeln.

Überraschenderweise konnten bei Kohlenwasserstoffen mit einem sehr niedrigen Ausgangsschwefelgehalt durch Adsorption der Schwefelverbindungen an Kupfer-, Silber- und Zink-haltigen Zeolithen Entschwefelungsgrade von weit über 90 % erzielt werden. Es gelingt sogar mit Hilfe des erfindungsgemäßen Verfahrens, Kohlenwasserstoffe bei einem entsprechenden Ausgangsschwefelgehalt bis auf einen Restgehalt von unter 5 ppb S zu entschwefeln.

Gegenstand der vorliegenden Erfindung ist daher ein Verfahren zur Feinentschwefelung von Kohlenwasserstoffen, die organische Sulfide ausgenommen Schwefelwasserstoff, Mercaptane und Thiophene, organische Disulfide, Trisulfide oder Tetrasulfide oder deren Mischungen enthalten, das dadurch gekennzeichnet ist, daß man diese Kohlenwasserstoffe mit Zeolithen, die mindestens 2 Gew.-% an Silber oder Zink oder deren Mischungen ausgenommen Cadmium enthalten, in Kontakt bringt, wobei die Zeolithe durch Ionenaustausch hergestellt und die ansgetauschten Zeolithe anschließend thermisch nachbehandelt werden.

Unter Feinentschwefelung ist hierbei die Entschwefelung von Kohlenwasserstoffen zu verstehen, die weniger als 20 Gew.-ppm Schwefel, vorzugsweise weniger als 10 Gew.-ppm Schwefel, enthalten.

Vorzugsweise enthalten die zu feinentschwefelnden Kohlenwasserstoffe 1 bis 20 Kohlenstoffatome.

Das erfindungsgemäße Verfahren kann sowohl in der Gasphase als auch in der Flüssigphase sowie kontinuierlich oder diskontinuierlich ausgeübt werden.

Die erfindungsgemäß verwendeten Zeolithe können z. B. in der Weise hergestellt werden, daß man die austauschbaren Kationen von Zeolithen ganz oder teilweise gegen kationen der Metalle Kupfer und/oder Silber und/oder Zink austauscht. Für diesen Ionenaustausch können z. B. Zeolithe der Typen A, X oder Y eingesetzt werden. Austauschbare Kationen sind beispielsweise Alkali- und/oder Ammoniumkationen.

Vorzugsweise beträgt der Gehalt des erfindungsgemäß verwendeten Zeolithen an Kupfer, Silber oder Zink jeweils mindestens 2 Gew.-%.

Der Ionenaustausch kann kontinuierlich, z. B. in einer mit Zeolith gefüllten Kolonne erfolgen, die von einer wäßrigen oder organischen Lösung von Kupfer- und/oder Silber- und/oder Zinksalzen und/oder -Komplexen durchströmt wird.

Ein diskontinuierlicher Ionenaustausch kann z. B. durch Zugabe des Zeolithen in diese Lösung erfolgen, die anschließend geschüttelt wird.

Vorzugsweise findet der Ionenaustausch bei pH-Werten von 5 bis 12 statt, was unter anderem von der Stabilität des ausgewählten Zeolithen abhängt. Für den Austauschprozeß günstige Temperaturen liegen zwischen 20 und 80 °C, die Austauschzeiten variieren zwischen einigen Minuten bis zu mehreren Stunden.

Bevorzugte Konzentrationen der Kupfer- und/oder Silber- und/oder Zink-haltigen Lösungen liegen zwischen 0,1 und 10 mol pro Liter, und bevorzugte Korngrößen der Zeolithe zwischen 0,01 und 5 mm.

Nach erfolgtem Austausch können schwach adsorbierte Kupfer- und/oder Silber- und/oder Zinksalze und/oder -Komplexe durch Extraktion mit einem geeigneten Lösungsmittel, wie z. B. Wasser oder einem Alkohol, vom Zeolithen entfernt werden.

Der ausgetauschte Zeolith wird bei ca. 100 bis 200 °C getrocknet und anschließend bei ca. 200 bis 600 °C thermisch nachbehandelt. Der fertige Kupfer- und/oder Silber- und/oder Zink-haltige Zeolith ist sowohl für die kontinuierliche als auch diskontinuierliche Feinentschwefelung von Kohlenwasserstoffen zu verwenden. Das erfindungsgemäße Verfahren kann in der Gas- oder Flüssigphase bei Temperaturen von 50 bis 350 °C, bevorzugt von 50 bis 130 °C, und bei Drücken von 1 bis 200 bar, bevorzugt von 1 bis 50 bar, durchgeführt werden.

Im weiteren kann das erfindungsgemäße Verfahren kontinuierlich bei einer "weight hourly space velocity"

$$\left( WHSV = \frac{\text{Menge Einsatzstoff}}{\text{Menge des Zeolithen} \cdot \text{Zeit}} \quad \left[ \frac{kg}{kg \cdot h} \right] \right.$$

von 0,05 bis 100 $h^{-1}$, bevorzugt von 1 bis 40 $h^{-1}$, ausgeübt werden.

Die Verfahrensbedingungen werden innerhalb obiger Grenzen in erster Linie durch die Wirtschaftlichkeit des Verfahrens festgelegt. So bestimmt z. B. die WHSV die Form der Durchbruchskurve und damit auch die Ausnutzung des Zeolithbettes bis zu der noch tolerierbaren Durchbruchskonzentration an Schwefelverbindungen.

Die Erfindung wird durch die folgenden Beispiele näher erläutert:

Die Schwefelgehalte der Kohlenwasserstoffe wurden gaschromatographisch bestimmt, wobei ein flammenphotometrischer Detektor und eine Chromatographie-Säule vom Typ CP-Sil 5[R] der Fa. Chrompak verwendet wurden.

Alle Prozentangaben bedeuten Gewichtsprozente. Dasselbe gilt für die Angaben in ppm und ppb.

Beispiel 1

500 g Natrium-Zeolith von Typ X wurden mit 750 ml einer 1 molaren $ZnCl_2$-Lösung versetzt, deren pH-Wert man zuvor mit konz. Salzsäure auf 5 eingestellt hatte. Die Suspension wurde 24 h bei 80 °C gerührt, der Zeolith anschließend bei 100 °C getrocknet und bei 300 °C thermisch nachbehandelt. Der Zn-Gehalt des Zeolithen betrug 6,23 %.

n-Butan mit einem Ausgangsgehalt von 60 ppb Schwefel in Form von Dimethyldisulfid wurde für 48 h kontinuierlich bei einer WHSV von 0,75 $h^{-1}$ und einer Temperatur von 100 °C bei einem Druck von 20 bar an dem oben beschriebenen Zn-Zeolithen bis auf einen Restgehalt von < 5 ppb S entschwefelt.

Beispiel 2

n-Butan, verunreinigt mit 150 ppb S in Form von Dimethyldisulfid, wurde kontinuierlich mit einer WHSV von 4 $h^{-1}$ vier Tage lang bei einer Temperatur von 120 °C und einem Druck von 50 bar über einen Kupfer-Zeolithen von Typ Y mit einem Cu-Gehalt von 4,5 % geleitet, der analog Beispiel 1 hergestellt worden war. Auch nach 4 Tagen lag der Schwefelgehalt des n-Butans noch unter der Nachweisgrenze von 5 ppb S.

Beispiel 3

Der Versuch wurde analog Beispiel 2 bei einem Druck von 9 bar durchgeführt. Auch in diesem Fall wurde n-Butan bis auf einem Schwefelgehalt von < 5 ppb S entschwefelt.

Beispiel 4

Ein Kupfer-Zeolith von Typ X mit einem Cu-Gehalt von 5,1 %, hergestellt analog Beispiel 1, wurde unter den in Beispiel 2 genannten Bedingungen getestet. Das n-Butan konnte auch in diesem Fall bis auf einen Schwefel-Gehalt von < 5 ppb S entschwefelt werden.

Beispiel 5

n-Butan mit einem Dimethyldisulfidgehalt, gerechnet als 2 ppm S, wurde bei einer WHSV von 3,75 $h^{-1}$, einer Temperatur von 120 °C und einem Druck von 30 bar 2 Tage kontinuierlich an einem Kupfer-Zeolithen vom Typ X mit einem Cu-Gehalt von 10 %, hergestellt analog Beispiel 1, bis auf einen Restgehalt von 20 ppb S entschwefelt.

Beispiel 6

Mit 500 ppb S in Form von Methyl-2-butylsulfid verunreinigtes n-Butan wurde mit einer WHSV von 1,5 $h^{-1}$ bein einer Temperatur von 60 °C und einem Druck von 20 bar über einen Zeolithen vom Typ X, hergestellt analog Beispiel 1, geleitet, der 6 % Zn und 4 % Cu enthielt. Nach einer Laufzeit von 48 h wurde eine Entschwefelung bis auf 43 ppb S erreicht.

Beispiel 7

Analog zu Beispiel 6 wurde ein Gemisch von $C_{20}$-Olefinen (Buten-Pentamere) entschwefelt. Der Restgehalt an Schwefel betrug 32 ppb S.

Beispiel 8

Ein Zeolith vom Typ X, hergestellt analog Beispiel 1, mit einem Ag-Gehalt von 10 % und einem Cu-Gehalt von 2 % wurde bei einer WHSV von 1,5 $h^{-1}$, einer Temperatur von 130 °C und einem Druck von 5 bar zur Entschwefelung von n-Butan mit einem Schwefelgehalt von 2 ppm S in Form von Methyl-1-butylsulfid (50 %) und Methyl-2-butylsulfid (50 %) eingesetzt. Nach einer Laufzeit von 36 Stunden betrug der Schwefelgehalt des n-Butans nur noch 27 ppb S.

Beispiel 9

Die Entschwefelung von n-Butan, das 150 ppb S in Form von Dimethyltrisulfid enthielt, wurde analog Beispiel 2 durchgeführt. Es konnte ein Restschwefelgehalt von < 5 ppb S, d. h. unterhalb der Nachweisgrenze, erzielt werden.

Beispiel 10

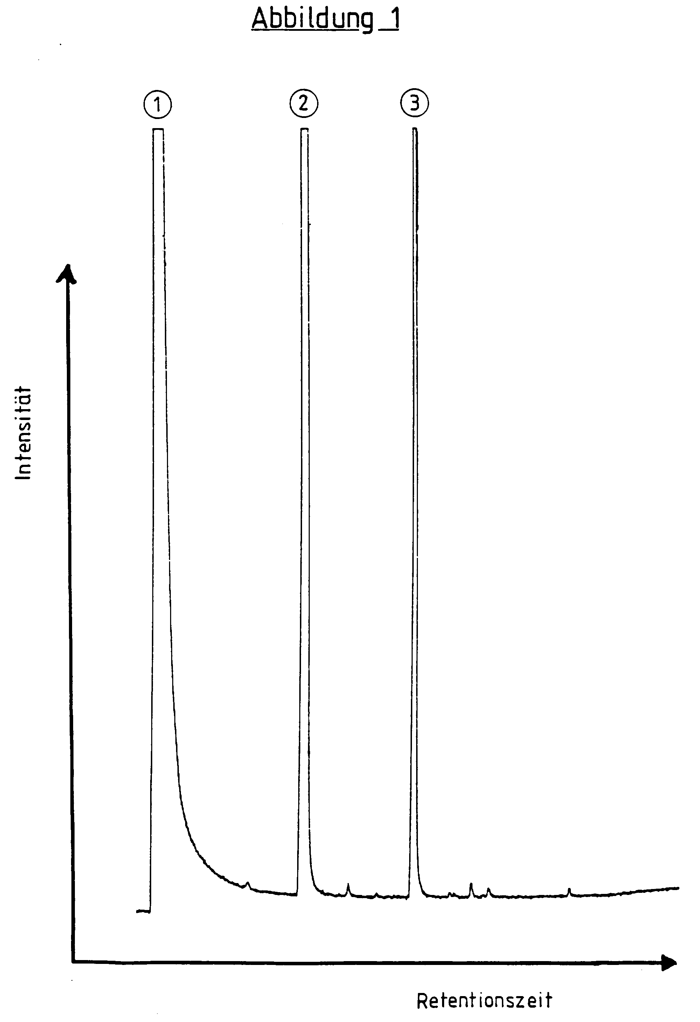

Ein Propen/Propan-Gemisch, bestehend aus ca. 75 % Propen und ca. 25 % Propan, das 646 ppb S in Form von Dimethyldisulfid und Dimethyltrisulfid enthält (Gaschromatogramm siehe Abb. 1), wurde mit einem Palladium-Hydrierkatalysator bei einer Temperatur von 20 °C und einem Druck von 15 bar in Kontakt gebracht.

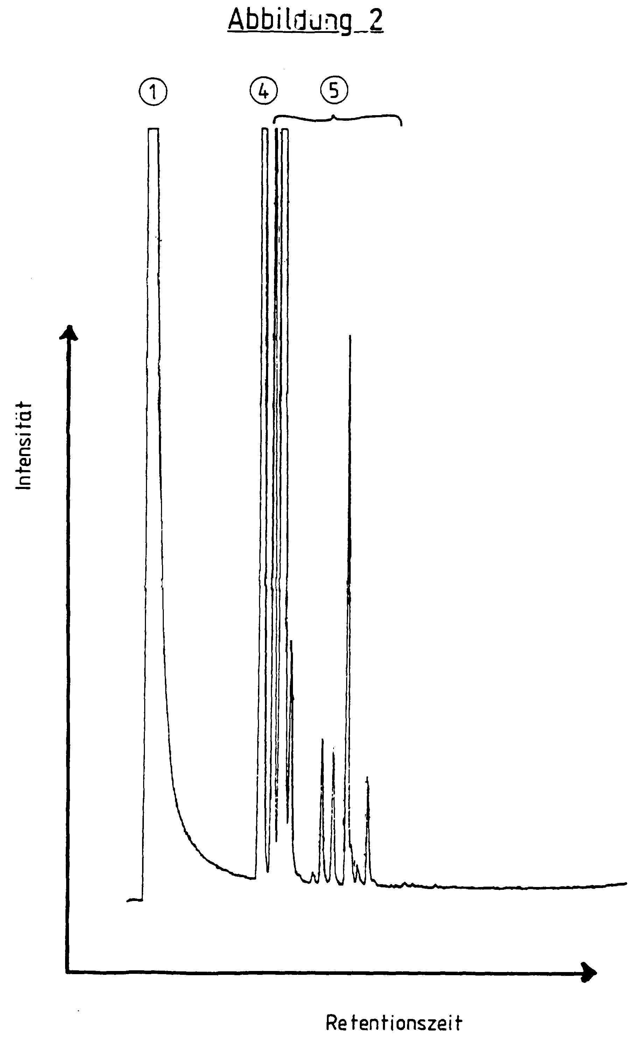

Hierbei wurden die beiden obengenannten Schwefelverbindungen in eine Vielzahl neuer schwefelhaltiger Verbindungen mit Methyl-propylsulfid als Hauptkomponente umgewandelt (Gaschromatogramm siehe Abb. 2).

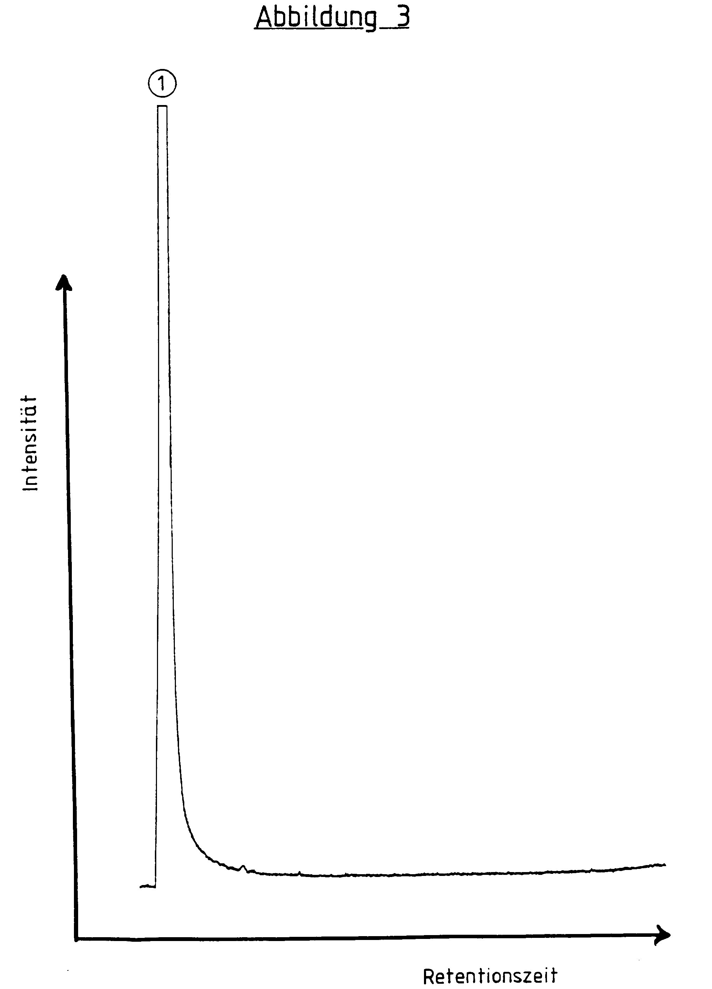

Dieses Reaktionsgemisch entschwefelte man 36 h lang an einem Zeolithen vom Typ X mit einem Gehalt von 10 % Ag und 2 % Cu, hergestellt analog Beispiel 1, bei einer Temperatur von 110 °C, einem Druck von 10 bar und einer WHSV von 20 $h^{-1}$. Das so erhaltene Propen/Propan-Gemisch enthielt nur noch einen Restschwefelgehalt von < 5 ppb S (Gaschromatogramm siehe Abb. 3).

Zu den Abbildungen 1 bis 3:

In den Abbildungen 1 bis 3 sind die Ergebnisse der gaschromatographischen Analysen wiedergegeben. Es ist jeweils die Peak-Intensität in Abhängigkeit von der Retentionszeit aufgetragen.

Abb.: 1 Gaschromatogramm des Propen/Propan-Ausgangsgemisches
Abb.: 2 Gaschromatogramm des Propen/Propan-Gemisches nach Kontakt mit dem Hydrierkatalysator

Abb.: 3     Gaschromatogramm des entschwefelten Propen/Propan-Gemisches

Die einzelnen Peaks in den Gaschromatogrammen wurden wie folgt identifiziert:

1   Propen/Propan
2   Dimethyldisulfid
3   Dimethyltrisulfid
4   Methyl-propylsulfid
5   verschiedene andere Schwefelverbindung

Vergleichsbeispiele

Vergleichsbeispiel 1 gemäß Shcherbina et al., Obshch. Prikl. Khim., No. 5 (1972), 113 - 114

Beispiel 2 wurde an einem nichtausgetauschten Natrium-Zeolithen vom Typ X wiederholt: Nach vier Tagen fand man nach dem Zeolithbett die gleichen Schwefelgehalte wie im Zulauf, und zwar ca. 150 ppb S.

Vergleichsbeispiel 2 gemäß Shcherbina et al., Obshch. Prikl. Khim., No. 5 (1972), 113 - 114

Beispiel 6 wurde an einem nichtausgetauschten Natrium-Zeolithen vom Typ X wiederholt: Wie im Vergleichsbeispiel 1 fand man auch hier einen vollständigen Durchbruch von Methyl-2-butylsulfid, und zwar in einer Konzentration von ca. 500 ppb, ger. als S.

Vergleichsbeispiel 3 gemäß JP 59/160 584

Beispiel 2 wurde an Aktivkohle als Entschwefelungsmedium mit einem Kupfer-II-oxid-Gehalt von 5,5 % wiederholt: Nach dem Entschwefelungsversuch enthielt das n-Butan noch ca. 85 ppb S.

Vergleichsbeispiel 4 gemäß DD-PS 241 196 und DD-PS 241 197

Beispiel 6 wurde an einem Mangan-Zeolithen vom Typ X mit einem Gehalt von 8 % Mangan wiederholt: Das n-Butan wies nach dem Entschwefelungsversuch den hohen Restgehalt von 350 ppb S in Forn von methyl-2-butylsulfid auf.

**Patentansprüche**

1.   Verfahren zur Feinentschwefelung von Kohlenwasserstoffen, die weniger als 20 Gew.-ppm Schwefel in Form von organischen Sulfiden, ausgenommen Schwefelwasserstoff, Mercaptane und Thiophene, organischen Disulfiden, Trisulfiden oder Tetrasulfiden oder deren Mischungen enthalten,
dadurch gekennzeichnet,
daß man diese Kohlenwasserstoffe mit Zeolithen, die mindestens 2 Gew.-% an Silber oder Zink oder deren Mischungen, ausgenommen Cadmium, enthalten, in Kontakt bringt, wobei die Zeolithe durch Ionenaustausch hergestellt und die ausgetauschten Zeolithe anschließend thermisch nachbehandelt werden.

2.   Verfahren nach Anspruch 1,
dadurch gekennzeichnet,
daß man die Kohlenwasserstoffe bei Temperaturen von 50 bis 350 °C feinentschwefelt.

3.   Verfahren nach den Ansprüchen 1 und 2,
dadurch gekennzeichnet,
daß man die Kohlenwasserstoffe bei Drücken von 1 bis 200 bar feinentschwefelt.

4.   Verfahren nach den Ansprüchen 1 bis 3,
dadurch gekennzeichnet,
daß die zu feinentschwefelnden Kohlenwasserstoffe 1 bis 20 Kohlenstoffatome enthalten.

## Claims

1. Method for the fine desulphurization of hydrocarbons which contain less than 20 ppm by weight of sulphur in the form of organic sulphides, excepting hydrogen sulphide, mercaptans and thiophenes, organic disulphides, trisulphides or tetrasulphides or mixtures thereof, characterized in that said hydrocarbons are brought into contact with zeolites which contain at least 2 % by weight of silver or zinc or mixtures thereof, excepting cadmium, the zeolites being prepared by ionic exchange and the exchanged zeolites then being posttreated thermally.

2. Method according to Claim 1, characterized in that the hydrocarbons are finely desulphurized at temperatures from 50 to 350°C.

3. Method according to Claims 1 and 2, characterized in that the hydrocarbons are finely desulphurized at pressures of 1 to 200 bar.

4. Method according to Claims 1 to 3, characterized in that the hydrocarbons to be finely desulphurized contain 1 to 20 carbon atoms.

## Revendications

1. Procédé de désulfuration fine d'hydrocarbures qui renferment moins de 20 ppm en poids de soufre sous forme de sulfures organiques, à l'exception de l'hydrogène sulfuré, des mercaptans et thiophènes, et des disulfures, trisulfures ou tetrasulfures organiques ou leurs mélanges, caractérisé en ce que l'on met en contact ces hydrocarbures avec des zéolites qui renferment au moins 2 % en poids d'argent ou de zinc ou leurs mélanges, à l'exception du cadmium, procédé dans lequel les zéolites sont produites par échange d'ions et les zéolites échangées sont ensuite traitées thermiquement.

2. Procédé selon la revendication 1, caractérisé en ce que l'on désulfure finement les hydrocarbures à des températures allant de 50 à 350°C.

3. Procédé selon les revendications 1 et 2, caractérisé en ce que l'on désulfure finement les hydrocarbures à des pressions allant de 1 à 200 bars.

4. Procédé selon les revendications 1 à 3, caractérisé en ce que les hydrocarbures qui doivent être finement désulfurés renferment de 1 à 20 atomes de carbone.

# Abbildung 1

# Abbildung 2

## Abbildung 3